(19) Europäisches Patentamt
European Patent Office
Office européen des brevets

(11) Publication number: 0 007 126 A1

# (12) EUROPEAN PATENT APPLICATION

(21) Application number: 79200343.6

(22) Date of filing: 27.06.79

(51) Int. Cl.³: C 07 C 15/085
C 07 C 2/66

(30) Priority: 06.07.78 US 922487

(43) Date of publication of application: 23.01.80 Bulletin 80/2

(84) Designated Contracting States: AT BE DE FR GB IT NL

(71) Applicant: UNION CARBIDE CORPORATION
270, Park Avenue
New York,N.Y. 10017(US)

(72) Inventor: Wolynic, Edward Thomas
15 Herkimer Road
Scarsdale New York 10583(US)

(72) Inventor: Bezman, Richard David
383 South Lexington Avenue
White Plains New York(US)

(72) Inventor: Rabo, Jule Anthony
19 Windmill Road
Armonk New York(US)

(74) Representative: Eggert, Hans-Gunther, Dr.
Oberländer Ufer 90
D-5000 Köln 51(DE)

## (54) Cumene synthesis process.

(57) Zeolite omega is found to be a unique zeolite-type catalyst for the alkylation of benzene with propylene to form cumene. At temperatures below 400°F., a cumene product containing less than 200 parts per million (weight) of the undesirable impurity n-propylbenzene is produced in high yield and without the unacceptably high rate of catalyst deactivation incurred with other zeolite catalysts.

EP 0 007 126 A1

Croydon Printing Company Ltd.

The present invention relates in general to the synthesis of cumene by the catalyzed reaction of benzene with propylene, and more particularly, the synthesis of cumene with the formation of minimal amounts of n-propylbenzene impurity. The synthesis process involves the reaction of benzene and propylene in the liquid phase in contact with a zeolite omega catalyst.

Isopropylbenzene or cumene, as it is commonly known, is currently produced in large volume principally as an intermediate in the production of phenol and acetone which are obtained by the acid catalyzed reaction of cumene with air. n-Propylbenzene impurity, which cannot be effectively removed from the cumene, produces propionaldehyde and can become further oxidized to the corresponding carboxylic acid. This leads to autocatalyzed acid activated side reactions that decrease the yield of the desired phenol and acetone. These organic acids are also corrosive toward the processing apparatus.

At impurity levels of the order of a few hundred parts per million, the autocatalytic and corrosion problems are not especially serious, but the formation of even the proportionally small amount of propionaldehyde in the acetone product is distinctly disadvantageous when this acetone product is used in the synthesis of organic resins such as polyepoxides. This is mainly because the aldehyde impurity carries through as an undesirable colorant.

Currently the most widely used commercial scale

cumene synthesis route is the catalytic condensation process using phosphoric acid on Kieselguhr as the heterogeneous catalyst. It is a liquid phase fixed bed process carried out at temperatures within the range of about 450 to 500°F., at a pressure of about 500-600 psig and using a benzene to propylene molar ratio of from 6 to 8. Although generally effective in producing a cumene product containing low amounts of n-propylbenzene, this advantage exacts a high price in equipment corrosion, and catalyst handling problems. The catalyst is itself highly corrosive and necessitates the use of expensive materials of construction for the reactor. Precisely controlled amounts of water must be added to the feed to prevent the irreversible conversion of orthophosphoric acid to the meta form with consequent rapid catalyst deactivation. The catalyst also has very low transalkylation activity resulting in a high concentration in the product of poly-substituted alkylaromatics. Additionally, upon deactivation, the non-regenerable catalyst undergoes significant agglomeration cuasing a tedious and time-consuming changeout procedure.

A less commonly used commercial alternative route to cumene synthesis uses homogeneous aluminum chloride/hydrochloric acid slurry catalyst. This process has problems of spent catalyst sludge disposal, the need for corrosion resistant materials of construction for process apparatus and complex catalyst-alkylate product separation equipment as well as poor single-pass cumene selectivity.

Because of the considerable success achieved in other hydrocarbon conversion processes using crystalline zeolite-based catalyst compositions, considerable effort has been expended by many in the field to develop a zeolite catalyst for use in cumene synthesis. Although in general, large pore molecular sieve zeolites have been found to catalyze both the direct alkylation of benzene with propylene and the transalkylation of diisopropylbenzene with benzene to form cumene, all of the prior proposed processes produce unacceptably high amounts of n-propylbenzene impurity at temperatures high enough to avoid rapid catalyst deactivation, or unacceptably rapid deactivation at temperatures low enough to avoid undue n-propylbenzene formation.

Accordingly, it is the principal object of the present invention to provide a cumene synthesis process using a zeolite-based catalyst in which both the cumene yield is high and the catalyst deactivation rate and the formation of n-propylbenzene are suitably low.

This object and others which will be apparent from the specification are accomplished by the process which comprises contacting in the liquid phase benzene and propylene in a molar ratio of at least 3.5:1 at a temperature of from 200°F. to 400°F. with zeolite omega, said zeolite omega having a $M_2O/Al_2O_3$ molar ratio of less than 0.20, preferably less than 0.05, wherein "M" represents alkali metal cations, and recovering the cumene reaction product thereby formed.

It is surprisingly found that zeolite omega is unique among crystalline zeolites of the molecular sieve type in catalyzing the alkylation of benzene with propylene. As a general class, the zeolite catalysts facilitate the formation of much larger proportions of n-propylbenzene than the aforementioned $P_2O_5$/Kieselguhr or $AlCl_3$/HCl catalysts under the same reaction conditions. Also the formation rate of n-propylbenzene over zeolite-based catalysts increases exponentially with increasing temperature. Accordingly, to retain the n-propylbenzene impurity in the cumene product at the commercial specification of below 200 ppm, the reaction temperature must be about 400°F. or less. With the exception of zeolite omega, the zeolite catalysts deactivate so rapidly under these temperature conditions that commercial operation is found to be altogether unfeasible. Zeolite omega, however, is found to have a remarkably high catalyst productivity before regeneration is required, a high reaction selectivity for cumene, an extremely low, essentially nil, selectivity for triisopropylbenzene, and to produce a cumene product containing less than about 200 ppm n-propylbenzene. Moreover, this level of performance is obtained without the costly feed pretreatment, complex refining train, expensive non-corrosive materials for reactor construction, and non-regenerable catalyst disposal problems that afflict the prior proposed processes.

Zeolite omega is described in detail and the method for its preparation disclosed in pending application

Serial No. 625,091, filed October 23, 1975, and is incorporated herein in its entirety by reference.

The composition of zeolite omega can be expressed stoichiometrically in terms of moles of oxides as follows:

$$1.0 \pm 0.5\ A_{\frac{2}{n}}O : Al_2O_3 : 5\text{-}20\ SiO_2 : 0\text{-}10\ H_2O$$

wherein "A" represents at least one cation having a valence "n", preferably a valence of 1 to 3 inclusive. The cations represented by "A" include non-metallic cations such as hydrogen, ammonium, and organic cations of the alkylonium type, particularly alkylammonium cations of the structure $R'_{(4-q)}\ H_q\ N^+$ wherein "q" has a value of from 0 to 3 inclusive and R' is an alkyl group, preferably methyl or ethyl, and also "A" can represent metal cations such as alkali metals, particularly sodium, alkaline earth metals, transition metals and rare earth elements of the lanthanide series. The transition metals are those having atomic numbers from 21 through 29, from 39 through 46 and from 72 through 78. The lanthanide or rare earth series comprises elements having atomic numbers from 57 through 71.

In addition to its chemical composition and in conjunction therewith, zeolite omega can be identified and distinguished from other crystalline substances by its X-ray powder diffraction pattern, the data for which are set forth below in Table A. In obtaining the X-ray powder diffraction pattern, standard techniques were employed. The radiation was the $K_\alpha$ doublet of copper, and a Geiger counter spectrometer with a strip chart pen recorder was used. The peak

heights, I, and the positions as a function of 2θ, where θ is the Bragg angle, were read from the spectrometer chart. From these, the relative intensities, and d(Å) observed, the interplanar spacing in Angstrom units corresponding to the recorded lines, were determined. In Table A, the more significant interplanar spacings, i.e., the d(Å) values which characterize and distinguish zeolite omega from other zeolite species and which must be present in the X-ray powder diffraction pattern of zeolite omega are set forth. The relative intensities of the lines are expressed as VS (very strong), S (strong), MS (medium strong) and M (medium).

TABLE A

| | Relative Intensity |
|---|---|
| 15.8 ± 0.4 | M |
| 9.1 ± 0.2 | VS |
| 7.9 ± 0.2 | M |
| 6.9 ± 0.2 | M |
| 5.95 ± 0.1 | M |
| 4.69 ± 0.1 | M |
| 3.79 ± 0.1 | S |
| 3.62 ± 0.05 | M |
| 3.51 ± 0.05 | M-MS |
| 3.14 ± 0.05 | MS |
| 3.08 ± 0.05 | M |
| 3.03 ± 0.05 | M |
| 2.92 ± 0.05 | MS |

Zeolite omega is readily prepared by digesting and crystallizing an aqueous mixture whose overall composition, expressed in terms of mole ratios of oxides, falls within the range of:

$$\frac{Na_2O + (Me_4N)_2O}{SiO_2} \quad \text{from about 0.1 to about 0.6}$$

$$\frac{(Me_4N)_2O}{(Me_4N)_2O + Na_2O} \quad \text{from} > 0 \text{ to about 0.6}$$

$$SiO_2/Al_2O_3 \quad \text{from about 5 to about 30}$$

$$\frac{H_2O}{(Me_4N)_2O + Na_2O} \quad \text{from about 10 to about 125}$$

where Me = $CH_3$.

Representative reactants are silica gel, silicic acid, colloidal silica, alkali metal silicate, and reactive amorphous solid silicas as the source of silicon, and activated alumina, gamma alumina, alumina trihydrate or alkali metal aluminate as the source of aluminum. Sodium hydroxide and tetramethylammonium hydroxide or halide are typical convenient sources of sodium and tetramethylammonium ions, respectively. The reaction system is formed by placing all the required reactants in the proportions hereinbefore defined in a reaction vessel and bringing the temperature of the system thus formed to a temperature of between 80 and 100°C. for 1 to 8 days.

An alternative synthesis method is disclosed in U. S. P. 3,923,639, issued to J. Ciric, December 2, 1975.

In that disclosure zeolite omega is denominated zeolite ZSM-4.

For use as a catalyst in the present invention the alkali metal cation content of the as-synthesized form of the zeolite is reduced to below 40 equivalent percent, and preferably below 5 equivalent percent, by conventional ion exchange techniques using aqueous acids when the alkali metal is to be replaced with hydrogen ions or aqueous solutions of salts of ammonia or metals other than alkali metals. The preferred form of the zeolite is the acid form in which not more than 10 percent of the $AlO_4^-$ tetrahedra are associated with metal cations and the remaining $AlO_4^-$ tetrahedra are associated with hydrogen cations or are not associated with any cation, i.e. are decationized. Acid sites can also be created by ion exchange with bivalent or multivalent metal cations. For steric reasons the tetramethylammonium cations present in the as-synthesized zeolite omega cannot be ion-exchanged, but these cations can be removed by thermal conversion to ammonium cations and then replaced by ion-exchange in the known manner. Decationization of these cation sites can be prevented during the thermal treatment by using a calcination environment comprising ammonia and nitrogen as disclosed in U.S.P. 3,853,743, issued December 10, 1974, to A. B. Schwartz.

The $SiO_2/Al_2O_3$ molar ratio of the zeolite omega catalyst is not critical and can coincide with the range of the as-synthesized zeolite, i.e. 5 to 20, provided only that below 6.4 the zeolite contains sufficient metal cations

to stabilize the structure against collapse if and when the zeolite is calcined at temperatures above 500°C. prior to use. The final catalyst zeolite form should have a degree of crystallinity sufficient to exhibit an adsorption capacity for oxygen at -183°C. and 100 mm.Hg of oxygen pressure of 12 weight percent based on the anhydrous weight of the zeolite. $SiO_2/Al_2O_3$ molar ratios in the range of 6.4 to 10 are much preferred.

A preferred process for preparing zeolite omega having a molar $SiO_2/Al_2O_3$ ratio of greater than 6.4 is crystallization from a gel having the following proportions:

$$Na_2O/Al_2O_3 = 3.00$$
$$[(CH_3)_4N]_2O/Al_2O_3 = 0.60$$
$$SiO_2/Al_2O_3 = 10.0$$
$$H_2O/Al_2O_3 = 144.$$

The gel is maintained at 95°C. for 90-100 hours. The zeolite product is then isolated by filtration, washed with water until the filtrate pH is less than about 10, and dried in air. Thermal removal of the tetramethylammonium cations is accomplished by the following heat treatment in a well-ventilated oven:

| Temperature | Rate or Time |
|---|---|
| Ambient to 350°C. | 10°C./min. |
| 350°C. | Hold for two hours |
| 350°C. to 550°C. | 10°C./min. |
| 550°C. | Hold for two hours |

Ammonium exchange of the calcined zeolite is conducted with three one-hour batch-wise reflux treatments using 10 percent $NH_4Cl$ solution followed by thorough water washing. Completion of the catalyst consists of preparing a mull compound of 80 percent (by weight) zeolite and 20 percent acid peptized alumina, extruding through a 1/16 inch die, drying the extrudate at 110°C. and activating at 550°C., preferably using the heating schedule used to remove the tetramethylammonium cations, supra.

The catalyst composition can consist of zeolite omega alone or in conjunction with diluents or binders which do not exhibit any adverse activity under the alkylation conditions involved. Gamma alumina serves particularly well as a binder-diluent in forming the extruded catalyst forms. Kaolinitic clays and inorganic oxides generally are also suitable. Additives may be used to render the binder or any amorphous phase formed upon activation of the omega catalyst inactive to the reactants. These may include $P_2O_5$ or other phosphorus compounds, both inorganic and organic.

The process can employ a fixed catalyst bed, a moving bed or a fluidized bed in the same manner as zeolite-based catalysts are employed in other well-known hydrocarbon conversion processes.

The two most critical factors in the present alkylation process are the alkylation temperature and the molar proportions of benzene and propylene in the feed composition. Catalyst productivity in terms of pounds

of cumene per pound of catalyst, raw material efficiency with respect to both benzene and propylene and product purity are all directly affected by the benzene/propylene molar ratio of the feed. Catalyst life and product purity with respect to n-propylbenzene are similarly affected by the alkylation temperature.

The production of undesirable n-propylbenzene has been found to be remarkably sensitive to the alkylation temperature employed. As is evident from the data set forth in Example 4, a peak temperature rise of only 25°F. from 395°F. to 420°F. using a benzene/propylene feed molar ratio of 7:1 and a benzene LHSV of 7.1 $hr.^{-1}$ results in an increase of n-propylbenzene formation from 100 ppm to 500 ppm in the cumene product. Accordingly, the alkylation temperature should be maintained below 400°F., and preferably at or below 395°F. Although n-propylbenzene formation generally decreases with decreasing alkylation temperature, the rate of deactivation of the catalyst is found to increase with decreasing temperature. For this reason, alkylation temperatures above about 200°F., and preferably above about 300°F. are employed. It will be understood that in adiabatic reaction systems, such as fixed bed reactors, the alkylation occurs over a range of temperatures resulting primarily from the exothermic nature of the primary alkylation reaction. The maximum temperature obtained in the bed can be controlled to a considerable degree by controlling the temperature of the feed mixture entering the bed, i.e. lowering the

temperature of the feed mixture decreases the peak temperature by providing a heat sink for the evolved heat of reaction. Thus, in the present process it is possible to carry out the alkylation reaction in such a manner that the bulk of the reaction takes place below 400°F. or even 395°F. while permitting some of the reaction to take place at 400°F. or higher. In such a process the formation of relatively large proportions of n-propylbenzene which occurs above 400°F. is balanced by the formation of very small amounts of this impurity at the lower temperatures. The resulting total cumene product of the reactions at all the various temperatures will, therefore, be commercially acceptable from the standpoint of purity. This type of process is considered to be within the scope of the present invention, but it is preferred to avoid even a peak temperature of 400°F. or higher.

The benzene/propylene feed molar ratio is most preferably at least 7 in order to take advantage of the very favorable catalyst productivity obtained at those levels. Even at a ratio of about 6, however, catalyst productivity is comparable or even superior to that of the present commercial catalysts. At ratios below about 5.0 both the benzene and propylene raw material efficiencies become unacceptably low for commercial operation. Consequently, the minimum benzene/propylene feed molar ratio should be at least 5.0, is advantageously at least 6, and is most preferably at least 7.

Pressure is not a critical process condition provided it is sufficiently high to maintain the reaction mixture in the liquid phase at the process temperatures involved. Similarly the benzene volume liquid hourly space velocity (LHSV) is not narrowly critical but is advantageously at least 2 $hr.^{-1}$, and preferably at least 5 $hr.^{-1}$.

The process of the present invention is illustrated in the following examples:

Example 1

(Preparation of Zeolite Omega Catalyst)

(A) In a solution of 160 g. (4.0 moles) NaOH in 300 ml. water was dissolved 156 g. (2.0 moles) $Al(OH)_3$. A solution of 89.9 g. (0.41 moles) tetramethylammonium chloride in 2580 ml. water was prepared. A 701.3 g. (10.5 moles) portion of hydrated amorphous silica was blended into this solution, after which the sodium aluminate solution was added slowly, and the resulting mixture stirred rapidly for several minutes. The viscous gel was sealed into a glass jar lined with polytetrafluorethylene and heated for 17 days at 95°C., during which time the crystalline zeolite settled to the bottom of the jar. The zeolite was collected on a Buchner funnel, and was washed repeatedly with water until the filtrate pH was less than 10. The washed product was dried at 110°C., and was then spread in a thin layer in an evaporating dish. The zeolite was placed into an air-purged oven, and was heated at 220°C. for 2 hours and at 525°C. for 4 hours to decompose the entrained organic material. The

chemical composition of the calcined product was:

| | |
|---|---|
| $Na_2O$ ................ | 2.8 wt-% (anhyd. basis) |
| $Al_2O_3$ ................ | 17.2 wt-% |
| $SiO_2$ ................ | 80.0 wt-% |

(B) The calcined zeolite of part (A) supra was subjected to 5 sequential one-hour batch-wise reflux ion exchange treatments using 10 ml. 10 percent $NH_4Cl$ per gram of zeolite per exchange. The product was washed until the filtrate was chloride-free, after which it was dried at 110°C. The composition of the ammonium exchanged zeolite was as follows:

| | |
|---|---|
| $Na_2O$ ................. | 0.02 wt-% (anhyd. basis) |
| $(NH_4)_2O$ .............. | 7.2 wt-% |
| $Al_2O_3$ .............. | 16.0 wt-% |
| $SiO_2$ .............. | 75.4 wt-% |

The dried ammonium exchanged zeolite was mixed thoroughly with acid-peptized boehmite alumina in order to produce a paste containing 80 wt-% zeolite and 20 wt-% alumina, both defined on an anhydrous basis. The paste was extruded to give 1/16 inch diameter pellets which were then dried at 110°C. The dried extrudate was converted to the acidic zeolite catalyst by spreading it in a thin layer in an air-purged oven and heating it for 2 hours at 220°C. and then 2 hours at 550°C. The product had a surface area of 320 $m^2/g$. (Nitrogen adsorption at -196°C. and $p/p_o$ = 0.03).

In testing the alkylation activity of the catalyst compositions of the present invention, a test unit having a

40-50 gal./day feed capacity was employed to permit catalyst evaluation at commercially realistic olefin mass velocities. This unit consisted of a 1.34 in. O. D. x 0.925 in. I.D. x 85 in. long stainless steel fixed bed reactor mounted in a three-zone furnace having independent zone temperature control. The reactor was equipped with a 0.25 in. O. D. axial thermowell housing a 90 in. moveable thermocouple which was used to precisely monitor the catalyst bed temperature profile and thus quantitatively track the deactivation front. A separate 1.25 in. O. D. x 1.00 in. I. D. x 30 in. long stainless steel tube filled with 6 x 10 mesh quartz chips and mounted in a one zone furnace served as a feed preheater. A propylene feed cylinder, a 55-gallon stainless steel benzene feed drum and a 55 gallon stainless steel alkylate product drum were mounted on scale platforms accurate to 0.1 lb. This allowed for precise feed rate monitoring and the calculation of run material balances and catalyst raw material efficiencies. The material balances on test runs proved to be excellent at 99.5 $\pm$ 0.5 percent. From the preheater the reactants entered the main reactor in an upflow mode. The reactor was typically loaded with 180 cc. of 6 x 10 mesh quartz chips (bed length = 17 in.) which served as a supplemental preheat section, 630 cc. of undiluted catalyst charge (bed length = 60 in.) followed by 85 cc. of 6 x 10 mesh quartz chips (bed length = 8 in.). After loading the reactor the following start-up procedure was employed.

The reactor was purged with 5 SCF/hr. of nitrogen for one hour at atmospheric pressure and ambient temperature. The catalyst bed was then heated to 400°F. and the nitrogen purge maintained for approximately 16 hours. The nitrogen purge was then stopped and the reactor was then brought to test pressure and prescribed inlet temperature under the prescribed benzene flow. After approximately one hour the propylene flow to the reactor was started and precise test conditions were established. Because of the highly exothermic nature of the alkylation reaction and the quasi-adibatic nature of the reaction system, a sharp reaction front ($\Delta T$ of about 80-100°F.) was set up in the catalyst bed. Catalyst deactivation occurred in a frontal rather than a homogeneous mode. Thus, periodic measurement of the reaction front position allowed for quantitative determination of catalyst deactivation rate expressed in in./hr. This is readily translated into a measure of catalyst productivity expressed in lb. cumene/lb. catalyst. In addition to catalyst productivity, the test unit provided quantitative measurement of catalyst activity, selectivity and regenerability at commercially realistic process conditions.

<u>Example 2</u>

Using the test unit and procedure as described above, a comparison of the catalyst life of the zeolite omega prepared in Example 1, supra, with the lives of three prior proposed zeolite-based catalysts and a $P_2O_5$/

Kieselguhr catalyst composition typical of those widely used commercially. The zeolite-based catalysts which were compared with zeolite omega were:

(a) A steam-stabilized zeolite Y (U.S.P. 3,130,007) prepared by ammonium ion exchanging a sodium zeolite Y having a Si/Al molar ratio of 2.5 to reduce the $Na_2O$ content to 2.2 wt-% and calcining same at a temperature of 600°C. for 1.25 hours in a flowing stream of steam at 14.7 psia. Ammonia produced by the thermal decomposition of the ammonium cations was continually removed from the proximity of the zeolite during calcination, the zeolite was quickly cooled in air to below 350°C. and then allowed to cool further to ambient room temperature. The product zeolite had an ion-exchange capacity of about 32 percent and a high degree of crystallinity as evidenced by an adsorption capacity for oxygen (-183°C. at 100 mm. oxygen pressure) of about 28 wt-%.

(b) A hydrogen form of mordenite having a $SiO_2/Al_2O_3$ molar ratio of 31, less than 0.02 wt-% $Na_2O$. Composition contained 20 wt-% alumina binder.

(c) A decationized form of zeolite L (U.S.P. 3,216,789) prepared by ammonium ion exchanging an as-synthesized form of zeolite L having the empirical chemical composition (anhyd):

$$.04\ Na_2O : 0.92\ K_2O : Al_2O_3 : 6.1\ SiO_2$$

to lower the $Na_2O$ content to $<$.02 wt-% and the $K_2O$ content

to 3.8 wt-% and thereafter calcining the alumina bonded ion-exchanged product in air at 500°C. for 2 hours.

The pressure in the reactor was maintained at 600 psig, the benzene/propylene feed mole ratio was 7:1, the benzene LHSV was 7.1 $hr.^{-1}$, the propylene mass velocity was 150 $lb./hr.-ft.^2$, and the catalyst charge was 630 cc. The catalyst life was considered to be co-extensive with the period the alkylation front remained in the reactor before breakthrough. The results are set forth in tabular form below:

| Catalyst | Alkylation Temp., °F. Inlet | Alkylation Temp., °F. Peak | Catalyst Life, Hrs. |
|---|---|---|---|
| Omega | 410 | 490 | 500 |
| | 370 | 450 | 500 |
| | 320 | 400 | 500 |
| | 300 | 380 | 500 |
| | 290 | 370 | 500 |
| Steam Stabilized Zeolite Y | 410 | 490 | 920 |
| | 390 | 470 | 330 |
| | 370 | 450 | 120 |
| | 360 | 440 | 50 |
| $P_2O_5$/Kieselguhr* | --- | 475 | 290 |
| $H^+$ - Mordenite | 295 | 375 | 100 |
| Decat. Zeolite L | 290 | 370 | 180 |

*Calculated from commercial operating data.

It is readily observed from the results obtained that the catalyst life of the zeolite omega charge was constant over the entire peak temperature range of 490°F.

to 370°F. whereas the conventional zeolite catalysts, as represented by the steam-stabilized zeolite Y composition, rapidly deactivated with decreasing temperature. Also, over the peak temperature range of 370°F, to 470°F. the omega catalyst was vastly superior to all other catalysts with respect to catalyst life.

Example 3

Using the same test unit and procedure as in Example 2, the performance of zeolite omega in catalyzing the alkylation of benzene with propylene to form cumene was evaluated with respect to productivity, selectivity, raw material efficiency and product purity at various benzene/propylene feed molar ratios. The reactions were run at a pressure of 600 psig, a peak temperature of 380°F., a propylene mass velocity of 50 lb./hr.-ft.$^2$, benzene/propylene molar ratios of 7:1, 5:1, 3.5:1 and 2:1 and benzene LHSV of 2.4 hr.$^{-1}$. The results are set forth in Table II on the following page:

TABLE II

| $C_6/C_3^=$ MOLAR RATIO | 7 | | 5 | | 3.5 | | 2 | |
|---|---|---|---|---|---|---|---|---|
| **PRODUCTIVITY:** | | | | | | | | |
| (lb. cumene/lb. catalyst) | 1080 | | 625 | | 454 | | 403 | |
| **SELECTIVITY: BENZENE UTILIZATION** | | | | | | | | |
| (mole %) | | | | | | | | |
| CUMENE | 93.1 | | 90.5 | | 86.7 | | 78.4 | |
| DIISOPROPYLBENZENE | 6.8 | | 9.4 | | 13.2 | | 21.2 | |
| TRIISOPROPYLBENZENE | 0.1 | | 0.1 | | 0.1 | | 0.4 | |
| **RAW MATERIAL EFFICIENCY:** | | | | | | | | |
| BENZENE, PROPYLENE (%)- | $C_6$ | $C_3^=$ | $C_6$ | $C_3^=$ | $C_6$ | $C_3^=$ | $C_6$ | $C_3^=$ |
| CUMENE + DIPB* + TIPB** | 97.62 | 92.42 | 97.01 | 90.03 | 95.41 | 87.65 | 90.91 | 76.98 |
| LIGHTS | 0.13 | 4.38 | 0.14 | 4.38 | 0.17 | 4.78 | 1.32 | 9.33 |
| HEAVIES | 2.25 | 3.20 | 2.85 | 5.59 | 4.12 | 7.57 | 7.77 | 13.69 |
| **PRODUCT PURITY:** | | | | | | | | |
| (ppm. n-propylbenzene/cumene) | 140 | | 280 | | 390 | | 1110 | |

*DIISOPROPYLBENZENE
** TRIISOPROPYLBENZENE

Example 4

Using the same test unit and procedure as in Example 2, a determination was made of the correlation of alkylation temperature with formation of n-propylbenzene for the zeolite omega of Example 1, the steam-stabilized zeolite Y of Example 2 and a rare earth exchanged form of decationized zeolite Y having 40 equivalent percent didymium cations, a $SiO_2/Al_2O_3$ molar ratio of 5 and a $Na_2O/Al_2O_3$ molar ratio of 0.15 (45 equivalent % $NH_4$ cations). In making the test runs a benzene/propylene feed molar ratio of 7, a benzene LHSV of 7.1 $hr.^{-1}$, a propylene mass velocity of 150 lb./hr. $ft.^2$, a pressure of 600 psig and a catalyst charge of 630 cc. were employed. The results are set forth in Table III below:

TABLE III

| Catalyst | Temp., °F. Inlet | Peak | n-propylbenzene in cumene product ppm (vol.) |
|---|---|---|---|
| Zeolite Omega | 310 | 380 | 20 |
| | 315 | 395 | 100 |
| | 320 | 400 | 120 |
| | 330 | 410 | 200 |
| | 340 | 420 | 480 |
| | 370 | 450 | 2900 |
| Steam Stabilized Y | 315 | 395 | 275 |
| | 320 | 400 | 300 |
| | 330 | 410 | 450 |
| | 340 | 420 | 600 |
| | 370 | 450 | 1400 |
| Rare Earth Exchanged Zeolite Y | 315 | 395 | 1200 |
| | 330 | 410 | 2000 |
| | 340 | 420 | 2500 |

Although the unique catalytic properties of zeolite omega are described hereinbefore with respect to the synthesis of cumene by direct alkylation of benzene with propylene, it will be understood that the present catalysts are also effective in catalyzing the transalkylation of benzene with di-and poly-isopropylbenzenes such as diisopropylbenzene and triisopropylbenzene. In fact, this transalkylation reaction occurs during the alkylation process between feed benzene and polyalklyated benzenes produced in situ in the reactor. In the transalkylation reaction the amount of n-propylbenzene produced is found to be quite small when utilizing the present catalyst compositions.

WHAT IS CLAIMED IS:

1. Process for preparing cumene which comprises contacting in the liquid phase benzene and propylene in a molar ratio of at least 3.5 : 1 at a temperature of from 200°F. to 400°F. with zeolite omega, said zeolite omega having a $M_2O/Al_2O_3$ molar ratio of less than 0.2 wherein "M" represents alkali metal cations, and recovering the cumene reaction product formed thereby.

2. Process according to claim 1 wherein the temperature of contact of zeolite omega with benzene and propylene is within the range of 200°F. to 395°F.

3. Process according to claim 1 wherein the temperature of contact of zeolite omega with benzene and propylene is within the range of 300°F. to 395°F.

4. Process according to claim 3 wherein the benzene and propylene are present in a molar ratio of at least 6.

5. Process according to claim 4 wherein the zeolite omega has a $SiO_2/Al_2O_3$ molar ratio of at least 6.4 and has a $M_2O/Al_2O_3$ molar ratio of not greater than 0.1.

6. Process according to claim 5 wherein the zeolite omega has a $SiO_2/Al_2O_3$ molar ratio of from 7 to 10, an oxygen adsorption capacity in the dehydrated state at a temperature of -183°C. and an oxygen pressure of

100 mm. Hg of greater than 17 weight percent, and has a $M_2O/Al_2O_3$ molar ratio of less than 0.07.

7. Process according to claim 5 wherein the benzene and propylene reactants are passed through a fixed bed reactor containing the zeolite omega catalyst at a benzene liquid hourly space velocity of at least 2 $hr.^{-1}$.

8. Process according to claim 7 wherein the benzene liquid hourly space velocity is at least 5 $hr.^{-1}$.

9. Process for preparing cumene which comprises contacting in the liquid phase benzene and propylene in a molar ratio of at least 3.5 : 1 at a temperature of from 200°F to 400°F with zeolite omega, said zeolite omega having a $M_2O/Al_2O_3$ molar ratio of less than 0.4 wherein "M" represents alkali metal cations, and recovering the cumene reaction product formed thereby.

European Patent Office

# EUROPEAN SEARCH REPORT

Application number

EP 79 200 343.6

## DOCUMENTS CONSIDERED TO BE RELEVANT

| Category | Citation of document with indication, where appropriate, of relevant passages | Relevant to claim |
|---|---|---|
| | GB - A - 1 506 429 (UNION CARBIDE) * claims 1 to 4, 10, 18 * | 1,5,6, 9 |
| | GB - A - 1 292 228 (BRITISH PETROLEUM) * claims 1, 3, 5 * | 1,7,8 |
| | US - A - 4 070 407 (W.O. HAAG et al.) * claim 1 * | 1-5 |
| | US - A - 3 385 906 (S. KAUFMAN) * claim 1 * | 1-3 |
| A | US - A - 3 631 120 (P.E. EBERLY JR. et al.) * claim 1, 7; column 5, lines 30 to 50 * | 1 |
| A | US - A - 3 641 177 (P.E. EBERLY JR. et al.) * claim 1; examples * | 1 |

CLASSIFICATION OF THE APPLICATION (Int. Cl.3)

C 07 C 15/085
C 07 C 2/66

TECHNICAL FIELDS SEARCHED (Int.Cl.3)

C 07 C 2/66
C 07 C 15/085

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 10-10-1979 | KNAACK |